(19) Europäisches Patentamt
European Patent Office
Office européen des brevets

(11) **EP 2 719 328 A1**

(12) **EUROPEAN PATENT APPLICATION**

(43) Date of publication:
16.04.2014 Bulletin 2014/16

(51) Int Cl.:
*A61B 5/00* (2006.01)    *A61B 17/34* (2006.01)
*A61B 19/00* (2006.01)

(21) Application number: **13187961.1**

(22) Date of filing: **09.10.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.10.2012 US 201213648517**
**18.04.2013 US 201313865650**

(71) Applicants:
• **Christie Digital Systems USA, Inc.**
**Cypress, CA 90630 (US)**
• **Christie Digital Systems Canada, Inc.**
**Kitchener, ON N2G 4Y7 (CA)**

(72) Inventors:
• **Zarei Mahmoodabadi, Sina**
**Waterloo, Ontario N2K 3N7 (CA)**
• **Wagner, Robert**
**Kitchener, Ontario N2G 4Y7 (CA)**
• **Pinho, George**
**Waterloo, Ontario N2V 2L9 (CA)**

(74) Representative: **Walker, Ross Thomson**
**Forresters**
**Skygarden**
**Erika-Mann-Strasse 11**
**80636 München (DE)**

(54) **Catheter discrimination and guidance system**

(57)    An infrared detector and image projector system is set forth comprising an IR imager/projector for capturing an image of a vein and a needle and projecting the image on a surface; and a discrimination and guidance system for discriminating the needle from the image, calculating parameters of the needle and based on these parameters causing the IR imager/projector to project the image of the needle and vein and additional information to assist in visualizing needle alignment with the vein and insertion through tissue into the vein.

FIG. 2

Printed by Jouve, 75001 PARIS (FR)

## Description

CROSS-REFERENCE TO RELATED APPLICATIONS

This application claims priority to US Patent Application 13/648,517 filed on October 10, 2012 and US Patent Application 13/865650 filed on April 18, 2013.

BACKGROUND OF THE INVENTION

1. Field of the Invention

The present invention is directed to medical imaging and more particularly to a catheter discrimination and guidance system for an infrared detector and image display system to assist clinicians in performing intravenous and other vein access procedures.

2. Description of the Related Art

Intravenous (IV) catheters are used to access veins for blood draw, and for fluid delivery. There are very few techniques for assisting clinicians in verifying a positive cannulation of a vein. The standard technique for peripheral IV access involves using a tourniquet to engorge veins, followed by palpation to identify a suitable vein and finally insertion of the catheter needle. Clinicians must rely on "feel" when inserting the needle into a vein and on observing blood flashback to ascertain when the catheter has successfully cannulated the vein. Statistics indicate that this trial-and-error process requires an average of 2.4 attempts and up to 20 min for a clinician to successfully cannulate a vein. Aside from the increased pain and anxiety experienced by patients, there are real costs associated with IV care. Patient throughput, nurse time, consumables, and increased infection rates all contribute to increased medical care costs for hospitals and governments.

Systems have been developed for assisting in venous access and overcoming the disadvantages of traditional trial-and-error techniques. One such system is the VeinViewer® infrared detector and image projector manufactured and sold by Christie Medical Holdings, Inc., a division of Christie Digital Systems, Inc., which is described in US Patent No. 7,239,909, and US Publication Nos. 2010051808, 20070158569 and 20060122515, the contents of which are incorporated herein by reference.

According to the VeinViewer® system, diffuse infrared light is used to image vasculature below the surface of the skin, and the image is then projected onto the skin to reveal the location of the vasculature. The vasculature image is projected in exactly the same anatomical location as the vasculature itself, and in its three-dimensional context (skin of patient) making it very easy to see the vessels. Also, since there is no transducer to hold, the clinician's hands are free to perform venous access.

Although the VeinViewer® system has been widely adopted by hospitals, its application has been somewhat limited by the fact that it cannot detect a successful cannulation event. Ultrasound is the only current visualization technology that can show if a successful cannulation has occurred. Ultrasound is commonly used for deep vein access such as PICC lines and CVC's, but is not typically used for peripheral veins.

Prior art relevant to the invention includes literature on the theory and mathematical modeling of the opto-characteristics of different materials, such as disclosed in:

1. *The optics of human skin.* Anderson, R.R. and Parrish, J.A. 1, 1981, The Journal of Investigative Dermatology, Vol. 77, pp. 13-19.

2. *Use of the Kubelka-Munk theory to study the influence of iron oxides on soil colour.* Barron, V. and Torrent, J. 4, 1986, Journal of Soil Science, Vol. 37, pp. 499-510.

3. *Optical properties of human skin, subcutaneous and mucous tissues in the wavelength range from 400 to 2000 nm.* Bashkatov, A.N., et al. 15, 2005, Journal of Physics D: Applied Physics, Vol. 38, pp. 2543-2555.

4. *Geometry related inter-instrument differences in spectrophotometric measurements.* Edstorom, P., et al. 2, 2010, Nordic Pulp and Paper Research Journal, Vol. 25, pp. 221-232.

5. *Light scattering at the boundary between two media.* Ivanov, A.P. and Barun, V.V. 1, 2011, Journal of Engineering Physics and Thermophysics, Vol. 84, pp. 23-32.

6. *New contributions to the optics of intensely light-scattering materials. Part I.* Kubelka, P. 5, 1948, Journal of the Optical Society of America, Vol. 38, pp. 448-457.

7. *An article on optics of paint layers.* Kubelka, P. and Munk, F. 1930, 1931, Zeitschrif fur technische Physik, Vol. 31, pp. 1-16.

8. *Anisotropic reflectance from tubid media. II. Measurements.* Neuman, M. and Edstrom, P. 5, 2010, Journal of the Optical Society of America, Vol. 27, pp. 1040-1045.

9. *Anisotropic reflectance from turbid media. I. Theory.* Neuman, M. and Edstrom, P. 5, 2010, Journal of the Optical Society of America , Vol. 27, pp. 1032-1039.

10. *Point spreading in turbid media with anisotropic single scattering.* Neuman, M., Coppel, L.G. and Edstrom, P. 3, 2011, Optics Express, Vol. 19, pp. 1915-1920.

11. *Analytic light transport approximations for volumetric materials.* Premoze, S. 2002. Proceedings of the 10th Pacific Conference on Computer Graphics and Applications. pp. 48-58.

12. *Optical properties of circulating human blood in the wavelength range 400-2500 nm.* Roggan, A., et al. 1, 1999, Journal of Biomedical Optics, Vol. 4, pp. 35-46.

13. *The finite element method for the propagation of light in scattering media; boundary and source conditions.* Schweiger, M., et al. 11, 1995, Americal Association of Physicist in Medicine, Vol. 22, pp. 1779-1792.

Also relevant to the present invention are prior art needle guidance systems, such as disclosed in:

14. *Biopsy needle detection in transrectal ultrasound.* Ayvaci, A., et al. 7, 2011, Computerized Medical Imaging and Graphics, Vol. 35, pp. 653-659.

15. *A novel method for enhanced needle localization using ultrasound-guidance.* Dong, B., Savitsky, E. and Osher, S. 2009. Proceedings of the 5th International Symposium on Advances in Visual Computing: Part I. pp. 1-9.

16. A motion adaptable needle placement instrument based on tumor specific ultrasonic image segmentation. Hong, J., et al. 2002. pp. 122-129.

17. *Localization of palm dorsal vein pattern using image processing for automated intra-venous drug needle insertion.* Kavitha, R. and Flower, L. 6, 2011, International Journal of Engieering Science and Technology, Vol. 3, pp. 4833-4838.

18. *Single camera closed-form real-time needle trajectory tracking for ultrasound.* Najafi, M. and Rohling, R. 2011. SPIE Proceedings of Visualization, Image-Guided Procedures, and Modeling. Vols. 7964, 79641F.

19. Methods for segmenting cuved needles in ultrasound images. Okazawa, S.H., et al. 3, 2006, Medical Image Analysis, Vol. 10, pp. 330-342.

20. *Near-infrared imaging and structured light ranging for automatic catheter insertion.* Paquit, V., et al. 2006. SPIE Proceedings of Visualization, Image-Guided Procedures, and Display. Vols. 6141, 61411T.

21. *Unified detection and tracking in retinal microsurgery.* Sznitman, R., et al. 2011. Proceedings of Medical Image Computing and Computer-Assisted Intervention. pp. 1-8

It is an object of an aspect of this specification to set forth a system for improving intravenous and other vein access procedures by guiding clinicians in achieving optimal intravenous (IV) placement while minimizing infiltration and extravasations effects.

SUMMARY OF THE INVENTION

According to the present invention, a catheter discrimination and guidance system is provided that detects the catheter and its related parameters from an acquired infrared image and uses these parameters to direct a clinician before and during the vein access procedure.

These together with other aspects and advantages which will be subsequently apparent, reside in the details of construction and operation as more fully hereinafter described and claimed, reference being had to the accompanying drawings forming a part hereof, wherein like numerals refer to like parts throughout.

BRIEF DESCRIPTION OF THE DRAWINGS

Figure 1 is a schematic representation of optical light and tissue interactions.

Figure 2 is a schematic representation of a catheter discrimination and guidance system, according to an embodiment of the invention.

Figure 3 is a flowchart showing operation of the catheter discrimination and guidance system in Figure 2.

Figure 4 is a schematic representation showing a calibration process of the preferred embodiment.

Figure 5 comprises three images illustrating steps in the catheter discrimination process, wherein Figure 5a is an initial catheter image, Figure 5b is the same image in the frequency domain, and Figure 5c shows the final discriminated catheter.

Figure 6 shows an image with line fit (Figure 6a) and the related intensity plot (Figure 6b) for an opaque catheter.

Figure 7 is a schematic representation showing a catheter with a clip-on marker, according to an additional embodiment.

Figure 8 is a flowchart showing a process for distinguishing catheters of different vendors using the clip-on marker of Figure 7.

Figure 9a is a schematic representation of the needle-tissue-vein interfaces and related physical parameters, and Figure 9b shows the needle-tissue-vein interfaces and related geometrical calculations.

Figure 10 is a further schematic representation of a catheter entering the tissue (Figure 10a) and passing through the tissue to penetrate the vein (Figure 10b).

Figure 11a is a schematic diagram of pre-insertion catheter alignment aided by a projected series of dots.

Figure 11b is a schematic diagram of pre-insertion catheter alignment aided by a projected compass line.

Figure 12 is a schematic representation of a vein midline (Figure 12a) and problems with catheter insertion off the midline (Figure 12b).

Figure 13a is a schematic diagram of pixel analysis to locate the series of dots of Figure 11a.

Figure 13b is a schematic diagram of pixel analysis to locate the compass line of Figure 11b.

DETAILED DESCRIPTION OF THE PREFERRED EMBODIMENTS

Prior to describing the principles of the present invention, it is helpful to briefly review the theory behind optical vascular imaging. Photon scattering in soft tissue is dependent on photon wavelength and tissue composition. Projecting an incident beam of diffuse light on tissue results partly in backscattered light that is detectable at the surface of the tissue, and forward-scattered light that travels through the tissue. The forward scattered light interacts with scattering sites within the tissue and loses its intensity due to elastic and inelastic scattering phenomena. The inelastic phenomenon is also known as absorption. The intensity of the backscattered light is dependent on the type of tissue at the point of observation.

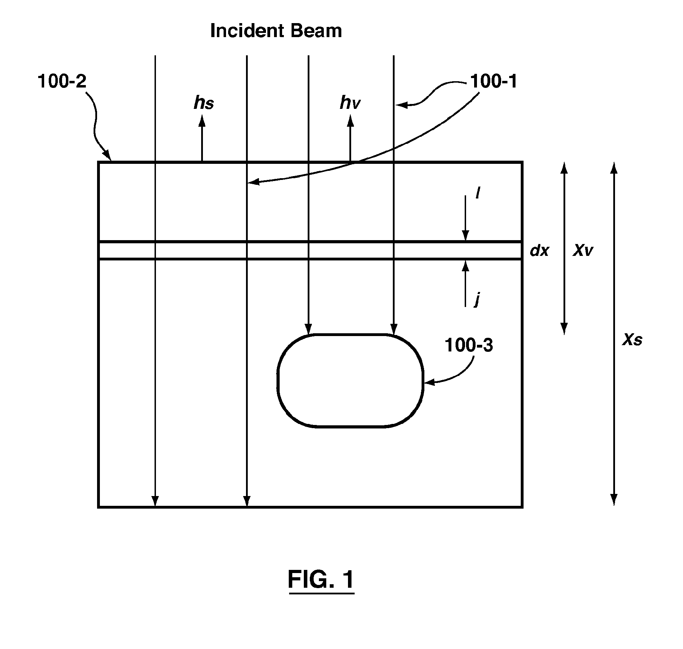

Turning to Figure 1, an incident light beam 100-1 of diffuse light intensity, $I$, is projected on a tissue medium 100-2 resulting in backscattered light of intensity of $J$ that is detected at the surface.

According to the known Kubelka-Munk theory of reflectance, a relationship exists, specifically a paired differential equation, between the incident and backscattered light and the tissue thickness. Solutions to the equation exist for different boundary conditions.

In order to estimate the depth $x_v$, under the skin, of a vein 100-3, three boundary conditions must be considered: (1) infinite tissue thickness, (2) tissue - vein boundary, and (3) needle - vein boundary.

Having regard to the first boundary condition, if the tissue is very thick (i.e. infinite tissue thickness), the incident beam 100-1 loses all of its intensity through scattering. Utilizing this condition, one can solve for all of the involved unknown parameters except for the scattering factor of the tissue.

At the tissue-vein boundary, almost all of the incident beam intensity is absorbed through elastic/inelastic scattering from blood within the vein, resulting in negligible reflectance. For a finite tissue thickness, there is no reflection at the exterior end medium (i.e. air). Using this condition, an equation can be arrived at for depth of the vein that is also irrespective of the tissue scattering parameter; however, it relies on the known thickness of the tissue where the total light absorption occurs.

Most light is absorbed within centimeters of the tissue thickness but this thickness value is unknown and its evaluation is not practical during performance of a clinical routine, due to various human tissue variants. This thickness dependency can be resolved using a predefined needle specific light reflectance over the skin and the measureable physical depth of the needle tip below the skin during the procedure. The needle depth can be measured for a standard catheter needle size through a procedure described in greater detail below.

This thickness dependency can also be resolved using the following methodology. The incident light intensity can be reduced to a level that substantially all photons are lost within a finite specific skin thickness. This skin thickness behaves like an infinite thickness under normal incident light application. In order to find this specific thickness, a vein contrast concept is used. Veins are discriminated within skin tissue due to the contrast resulting from significant light absorption in veins. Contrast is lost if the photons are not able to reach the vein surface due to lack of light intensity and the scattering/absorption effects only within skin tissue. Thus, light intensity is reduced to a level at which vein contrast is lost and, accordingly, photons annihilate right at the vein surface (the vein depth) and the skin thickness can be considered infinite in practical application. Measuring the related light intensity and reflection assists in resolving undefined tissue-related parameters.

In order to determine the needle-vein intersection (i.e. when the needle tip touches the vein surface, consecutive values of needle reflectance below the skin are registered during the catheter insertion process. It will be noted that, due to the high absorption of blood, the same needle reflectance values are expected to be detected at the needle tip (observed over the skin) while it is in the vein. Accordingly, needle-vein intersection occurs when the observed needle tip reflectance becomes equal to vein reflectance.

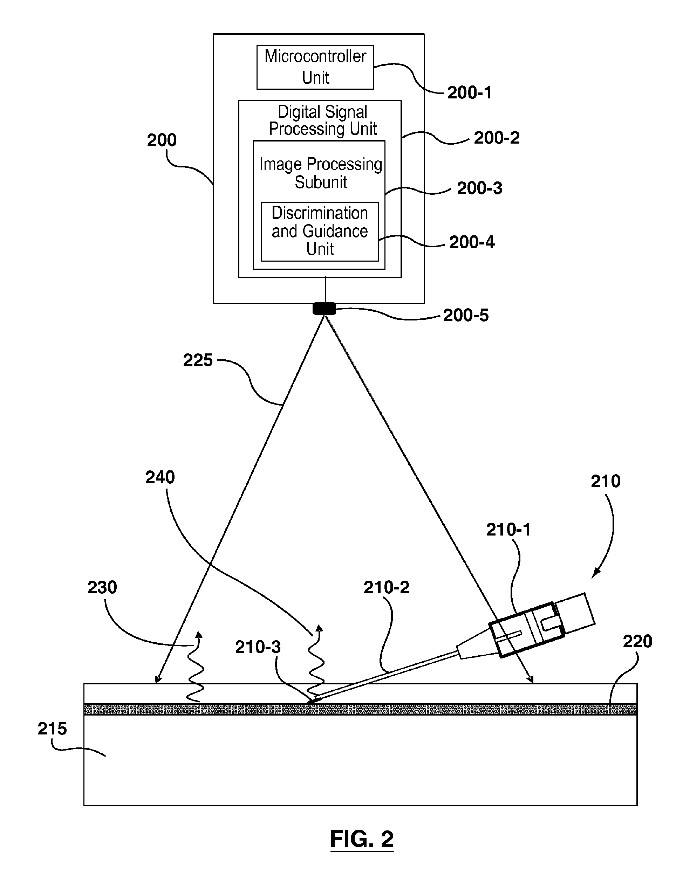

Turning to Figure 2, a catheter discrimination and guidance system 200 is shown that, according to an embodiment of the invention, is incorporated into an infrared detector and image projector system, such as the VeinViewer® system manufactured and sold by Christie Medical Holdings, Inc. The catheter discrimination and guidance system 200 includes three main units: a microcontroller 200-1 for controlling overall operation of the system, a digital signal processing unit 200-2 for performing mathematical calculations and estimations, and an IR imager/projector 200-5, with associated imaging optics. The digital signal processing unit 200-2, in turn, includes an image processing subunit 200-3 for performing image enhancement operations and, optionally audio signal generators for generating audio signals via a speaker 200-6, as discussed below. The catheter discrimination and guidance unit 200-4 is a sub-unit of the image processing subunit 200-3, and is used for discriminating and identifying a catheter system 210 from input images taken by the system 200 and guiding the clinician during intravenous procedures, as discussed in detail below.

The exemplary catheter system 210 depicted in Figure 2 is of conventional design, including a catheter housing 2 10-1 terminating in a catheter 210-2 and needle 210-3, used to insert the catheter into soft tissue 215 having a vein 220.

IR light 225 from IR imager/projector 200-5 illuminates the patient's soft tissue 215 with light in the range of 850 +/- 40nm, which is mainly absorbed by blood (haemoglobin) in the vein 220. The unabsorbed light 230 is reflected back and is detected by the IR imager/projector 200-5. The reflected image is processed by the image processing subunit 200-3, and then re-proj ected on the patient's skin in real-time to show the location of vein 220 beneath the patient's skin. The IR imager/projector 200-5 preferably includes a cut-off filter and a polarizer to differentiate reflected imaging light from background IR light in the environment.

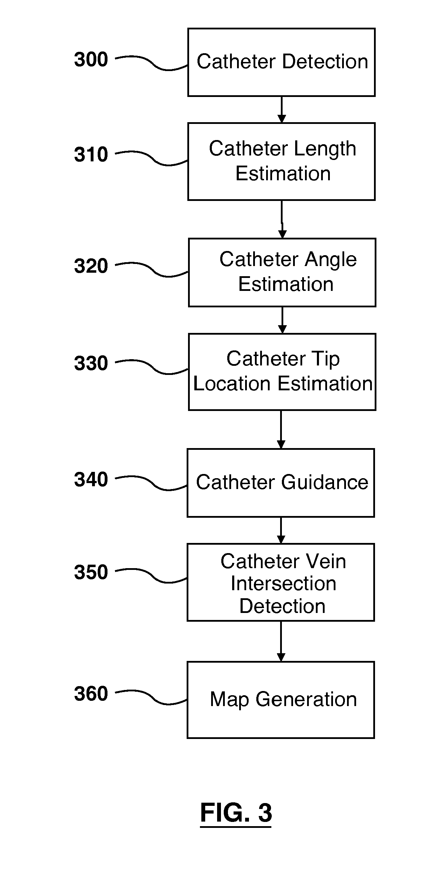

With reference to Figures 2 and 3, during an intravenous process, the clinician brings the catheter 210-2 within view of the IR imager/projector 200-5. At step 300, the catheter discrimination and guidance system 200-4 discriminates the catheter and the underlying needle 210-2 from the background image utilizing their specific opto-characteristics 210-2.

The catheter outline is detected and the device observed catheter length is determined at step 310. The catheter length is the observed distance between the catheter tip to the catheter housing, which are discriminated accordingly. The detected length value is used to calculate the insertion angle of the needle and locate the tip of the needle as it is inserted into the skin and as it penetrates the vein. Since the system 200 captures a two dimensional view of a scene, the actual length of the catheter 210-2 is different from its device observed value but is related to it by the angle it makes with the skin surface and can be determined using perpendicular projection, as described below. The maximum observed length is the actual length of the catheter and can be estimated according to a calibration process described below. Finally, the location of the catheter start and end points are determined (i.e. the point that connects the catheter to the catheter housing is the start point and the free end of the catheter is the end point), as discussed below.

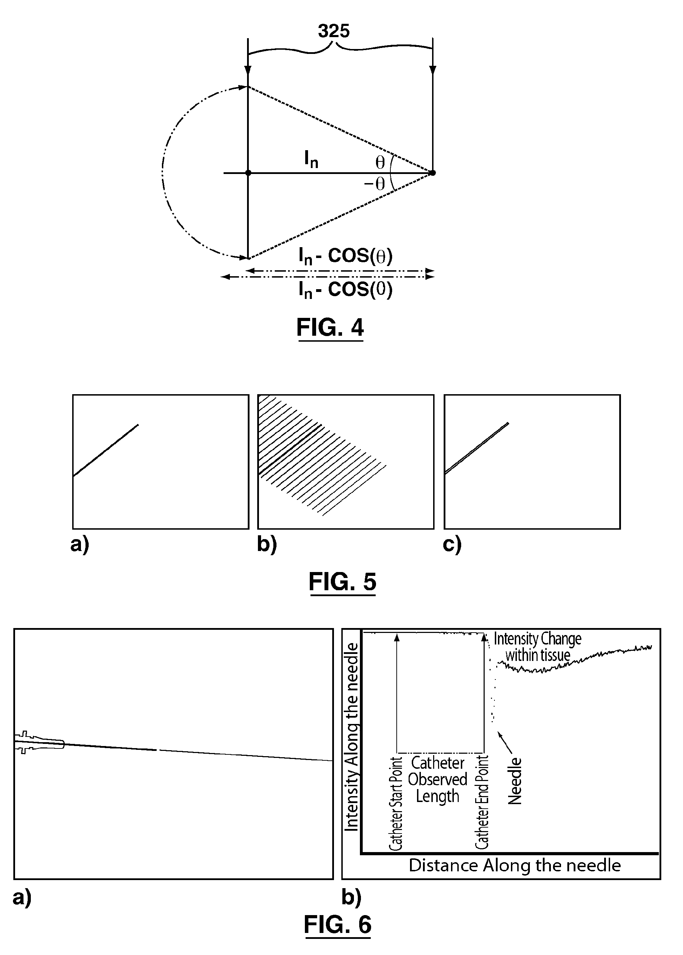

Since catheters are gauged according to their expected usage and come in various sizes, the calibration process permits calculation of the length for various catheter sizes without requiring any external predefined input from the user. The actual length of the catheter 210 is closely related to the length value as observed by the IR imager/projector 200-5 by the angle it makes with the skin surface at the point of vein access. Therefore, in use the clinician first follows a calibration process by which the catheter is positioned under the IR imager/projector 200-5 of system 200 and is rotated through small positive and negative angles relative to the skin surface, passing through zero degrees, in order to register and measure the actual catheter length, illustrated schematically in Figure 4, where the actual length of the catheter (maximum observed value) is observed when the catheter is oriented at an angle of zero degrees to the skin surface and the IR imager/projector 200-5.

The system 200-4 must discriminate the catheter from its environment. The catheter discrimination process is slightly different depending on whether the catheter is translucent or opaque. In either case, multi-resolution analysis is performed to expedite the catheter and needle discrimination processes, whereby captured images are first reduced to a lower resolution and an initial analysis is performed. Later the remaining analyses (e.g. frequency filtering, length and angle measurements) are performed using the original-size images.

Translucent catheters are composed of material that is permeable to infrared light and accordingly not visible to the IR imager/projector 200-5. However, by using a physical polarizer with the IR imager/projector 200-5, the catheter appears as a solid black line of measurable thickness in the captured image (Figure 5a). Next, the catheter discrimination and guidance system 200-4 applies a frequency filter to the image and analyses the image to locate the pixel having minimum intensity value (Figure 5b). This pixel is used as a 'seed point' to find catheter contour pixels with similar intensities and thereafter to estimate the length of the needle, as described below.

An opaque catheter has different characteristics than a translucent catheter, and is visible in the infrared wavelength region. The opaque catheter covers the metallic needle and totally absorbs the infrared light. The detection process for opaque catheters is similar to the process for translucent catheters discussed above, but the seed point is chosen as the point having maximum intensity value (rather than minimum intensity value). A small length of the needle (about 1-2mm) is uncovered at the tip of the catheter and its length is measured using the procedure described earlier for translucent catheters.

The image is later thresholded about the intensity of the seed point, thereby allowing a range of intensities. However, as a result of catheter positioning, angle, absorption and other factors involving scattering, it is not possible through the use of simple thresholding to obtain a solid line representing the catheter. Therefore, as shown in Figure 6a, a line-fit is estimated from the available catheter points and later superimposed over the catheter. The catheter start point is identified by detecting a significant width change in the housing along the line-fit, where the catheter starts. Figure 6b shows the intensity change along the superimposed line in Figure 6a. From Figure 6b, it will be noted that the intensity change along the catheter observed length is almost constant and is delimited by an exponential transition at the catheter end point. The catheter observed length can be calculated from the catheter start point (i.e. housing width change shown in Figure 6a) and catheter end point (i.e. exponential transition in intensity shown in Figure 6b). An abrupt intensity drop beyond the catheter end point indicates the tip of the needle, as shown in Figure 6b. The needle- vein intersection occurs when needle enters the vein, and is indicated by disappearance of the abrupt intensity drop.

As an alternative to catheter discrimination based on detecting the location of the catheter start point at the catheter housing 210-1, opto-genic catheters may be used. This alternative is especially useful with non-standard catheters whose length does not fit within the field of view of the system 200. Specifically, opto-genic catheters may be provided with optical markings that can be detected using image processing algorithms and used to estimate the visible length of the catheter (i.e. from the marking to the catheter tip). Opto-genic markings can be made using an etching process on the catheter to change its optical characteristics (e.g. specular reflection). The markings do not hamper the hygienic or normal clinical functionalities of the catheters.

Where opto-genic catheters are not available, a clip-on marker 210-4 may be provided, according to an additional aspect of the invention, to assist in discriminating different catheter types that are not compatible the preferred catheter discrimination and guidance system 200 (i.e. the Vein Viewer ® system, discussed above). The marker 210-4 is attached to or removed from the catheter housing 210-1 by a simple clip-on arrangement. The length and end point features of the marker are identified using the image processing algorithms already set forth herein, followed by the angle measurement and calibration process set forth herein.

The marker 210-4 is designed so as not to hamper the normal functionality of the catheter 210. The markings can include security-enabled, manufacturer-specific features that can be identified using the image processing routines set forth herein.

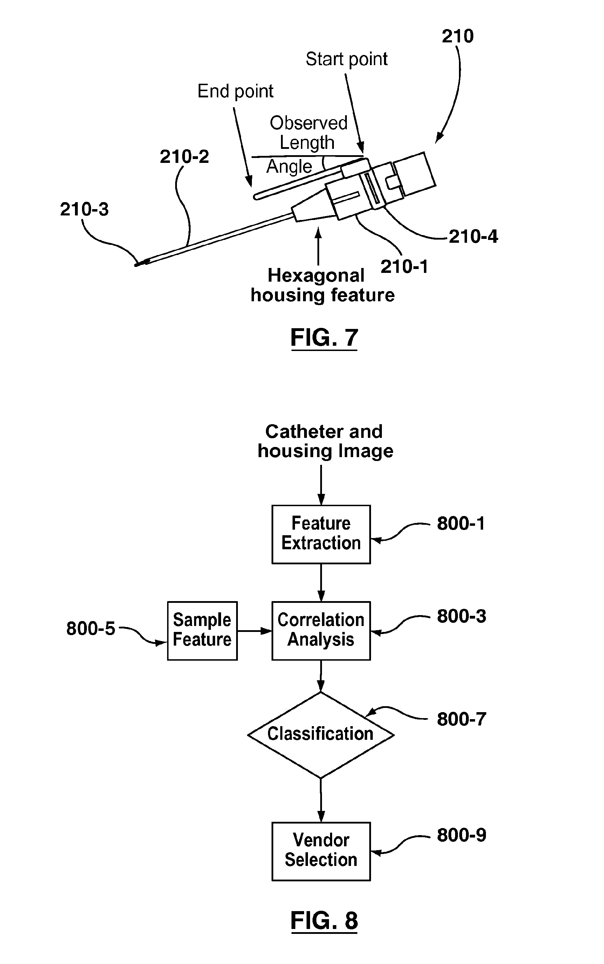

With reference to Figure 8, catheters manufactured by different vendors can be successfully distinguished based on a geometric correlation analysis of catheter housing geometrical features. In operation, a feature extraction process is performed (800-1) on the housing image captured using the system 200 to discriminate vendor-specific geometrical features of the housing (e.g. the representative hexagonal housing feature shown in Figure 7). Next, a correlation analysis is performed (800-3) between the extracted feature and a priori sample features (800-5) provided by the different vendors. The analysis identifies the sample feature having high correlation ratio, classifies it (800-7) and associates the extracted feature with a vendor class (800-9).

Although the marker set forth herein is described as being of added clip-on design, a person of skill in the art will understand that the marker can be molded in as part of the catheter housing.

Returning to Figure 3, at step 320, the system 200-4 determines and updates the angle that the catheter 210 makes with the skin surface, then at step 330, the system locates and updates the tip of the catheter by detecting backscatter light 240.

At this stage, the required catheter-related values (e.g. its length, angle and tip) have been determined and the IR imager/projector 200-5 then projects images and information onto the skin in order to assist the clinician in visualizing the catheter insertion process while the catheter is partly in the skin and out of view (step 340) and informs the user when the catheter and needle enter the vein (step 350), as discussed further below.

Finally, at step 360, the IR imager/projector 200-5 generates and projects a map (location and depth) of veins in close proximity to the vein access point, irrespective of the type and color of the skin. No tissue characteristic parameters have to be known a priori.

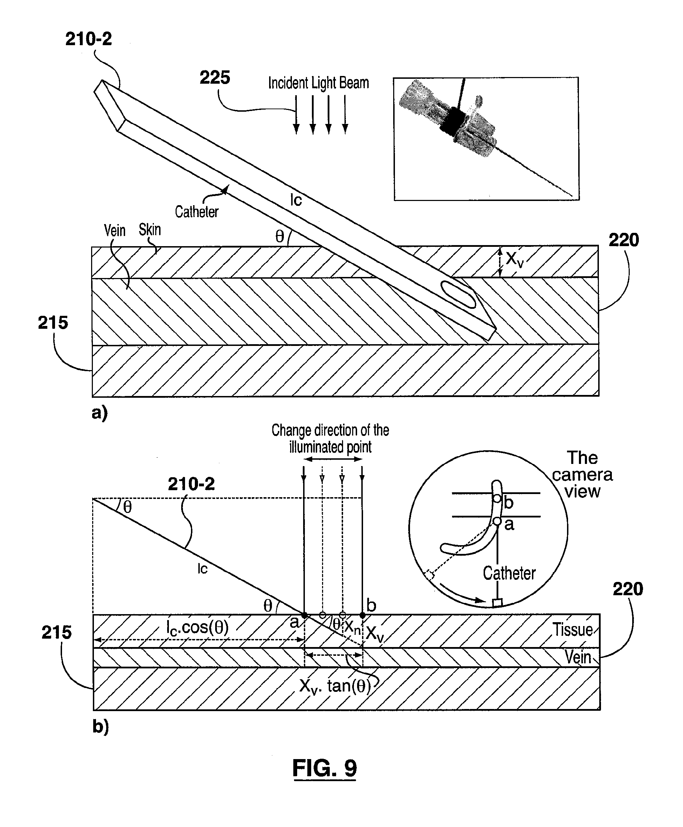

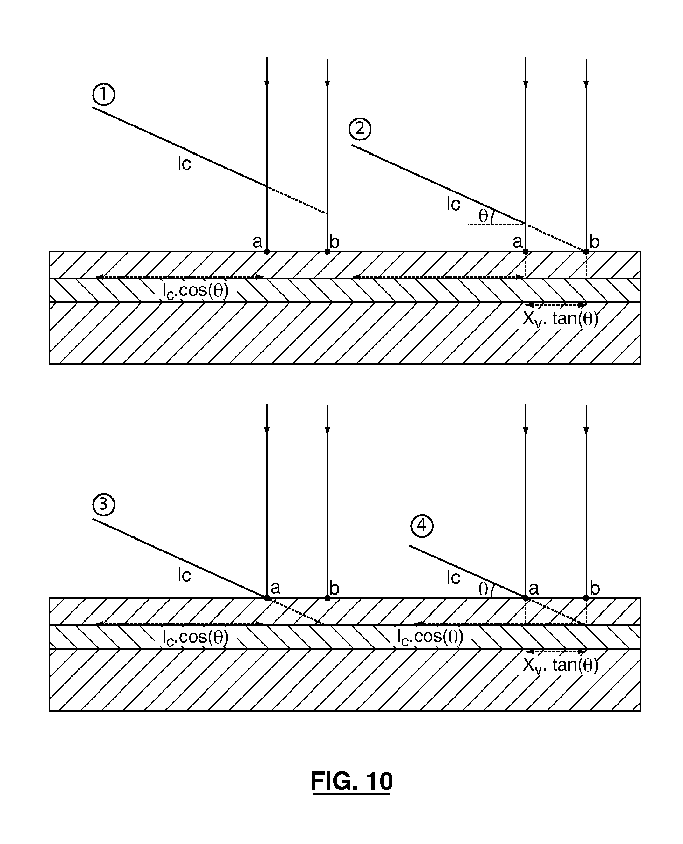

Figures 9 and 10 are schematic representations showing implementation of the process in Figure 3, in a clinical application. In particular, Figure 9a is a schematic representation of the catheter-tissue-vein interfaces and related physical parameters, and Figure 9b shows the catheter-tissue-vein interfaces with geometrical calculations for needle length, $ln$, vein depth, $x_v$, and angle, $\theta$, between the needle and the skin surface.

The critical vein depth value can be evaluated, as described previously, and the catheter positioning angle can be calculated from the catheter observed length based on perpendicular image acquisition and projection:

$$(1.1) \qquad l'_c = l_c.cos(\theta)$$

where $l'_c$ is the value observed at the IR imager/projector 200-5. At the start of the clinical procedure, the catheter tip is at the skin surface of the tissue at point $a$ (the catheter - skin access point). When the tip penetrates the skin to the vein surface, it travels from point $a$ to point $b$ (the needle - vein access point) on the skin surface: Where point $b$ can be calculated using the procedure explained earlier using the needle reflectance and its tip location.

Thus, at the vein surface:

$$(1.2) \quad |\vec{b} - \vec{a}| = |\vec{ab}| = x_v.tan(\theta)$$

Figure 10 shows needle insertion from above the skin (Figure 10a) to the vein surface (Figure 10b). When the needle enters the field of view of the IR imager/projector 200-5, its end points are automatically detected, as described above. From the detected catheter length, the angle, $\theta$, that the catheter makes with the skin surface is calculated from its observed length, and thereafter points $a$ and $b$ are located and projected. In particular, from point $a$ (the intersection of the needle and the skin), point $b$ is determined from the estimated vein depth, $x_v$.

The 200-5 can be configured to reduce light intensity until vein contrast is lost in order to determine an estimate for the vein depth, $x_v$, as discussed above. As tissue characteristics may influence vein depth, this can be performed as a short calibration process prior to step 340.

Thus, according to step 360, the system 200-4 causes IR imager/projector 200-5 to project the points *a* and *b* on the skin surface (e.g. using high intensity coloured dots) so that, the clinician is guided to adjust the direction of the catheter from the insertion point *a* in a straight line following the vein to point *b,* as shown in the inset portion at the top right of Figure 8b. In order to help the clinician to visualize the process, two lines are drawn perpendicular to the direction of approach at these two points. In addition to projecting high intensity dots and lines, it is contemplated that additional supplementary visual or audio information may be provided to indicate venous puncture (e.g. an audio signal via speaker 200-6, or color change alerts).

Lateral targeting can also be provided for the catheter before the catheter tip penetrates the skin surface. The catheter discrimination and guidance system 200 can be configured to provide guidance to a clinician as to the location of a suitable vein and a suitable direction in which to insert the catheter prior to insertion.

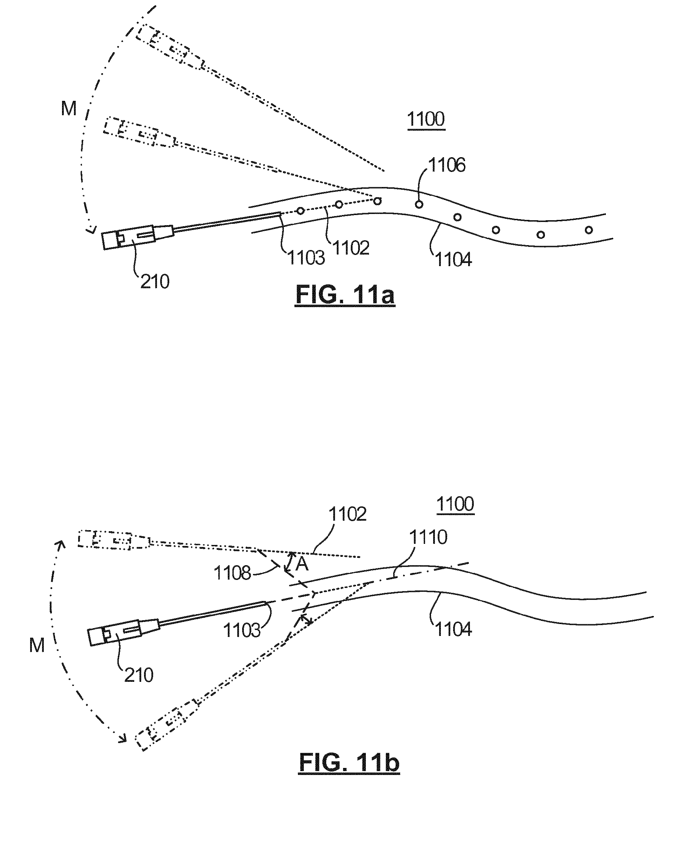

Figure 11a shows the catheter 210 over a skin surface 1100, onto which is projected a catheter direction line 1102 representative of the catheter 210 direction in the plane of projection. The catheter direction line 1102 can be determined as discussed above with respect to Figures 6a and 6b. In this embodiment, the line 1102 extends past the tip 1103 of the catheter 210 a predetermined distance, so as to visually indicate the direction of the catheter needle. The discrimination and guidance unit 200-4 tracks the catheter 210 and projects the line 1102 as the catheter 210 is moved, as indicated by arrow M.

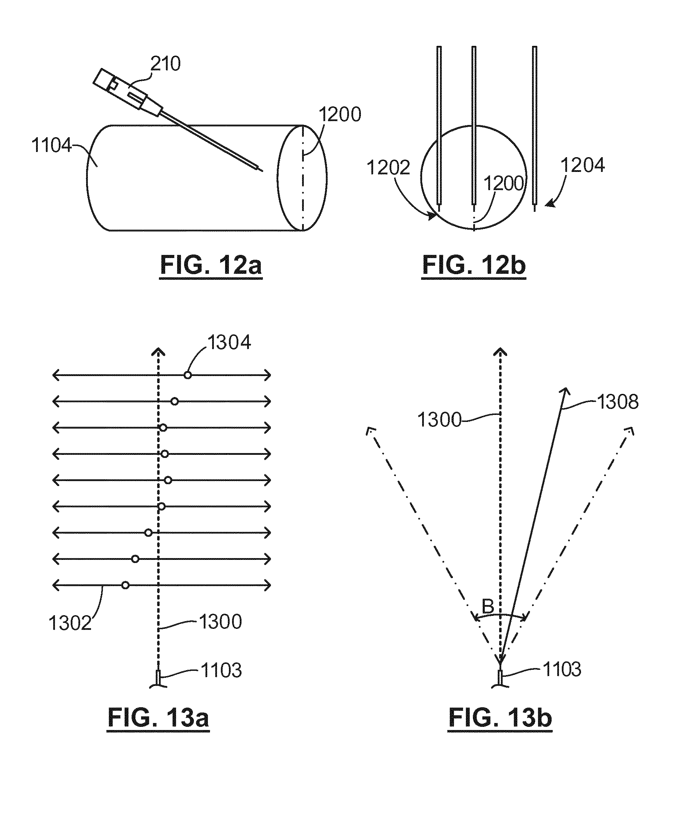

Also projected is a vein path indicator for a vein 1104. In this embodiment, the vein path indicator includes a series of dots 1106. The dots 1106 are located along the vein's midline 1200, as shown in Figure 12a, because even a slight deviation may result in an unwanted second puncture 1202 (Figure 12b) of the vein leading to intra/extravasations or may result in missing the vein entirely, at 1204. The dots 1106 are not limited to the circular dots illustrated, but may be rectangular or other shape.

The discrimination and guidance unit 200-4 is configured to determine the closest vein(s) to the catheter 210, and project the midline of each of such veins as a series of dots 1106. Signal conditioning for the exact locations of the points is performed to provide a consistent and nonvolatile presentation. That is, a filter can be applied to calculated point locations, so that visible jitter or insignificant positional fluctuation of the points is avoided.

Images captured for veins tend to be darkest at vein midlines, as more light is absorbed by a vein at its midline due to the midline typically being the thickest part of the vein. To generate the series of dots 1106, the discrimination and guidance unit 200-4 is configured to perform a process for points along a tracking line 1300 which is coincident with the catheter direction line 1102 and extends from the catheter tip 1103 and up to a predetermined length forward in the captured image, as shown in Figure 13a. The process may be performed for every pixel along the tracking line 1300.

The process includes analyzing image pixels of a series of spaced-apart lateral lines 1302 perpendicular to the tracking line 1300. The lateral lines 1302 symmetrically extend to both sides of the tracking line 1300 for a predetermined distance. The lateral lines 1302 may be evenly spaced at a predetermined interval. For each lateral line 1302, the discrimination and guidance unit 200-4 performs a search to find one or more sufficiently dark pixels 1304 and treats these pixels as vein midpoints. The process can determine that a pixel is sufficient dark when the pixel's intensity does not exceed a predetermined intensity. The acquired vein midpoints are then projected over the skin using a specific color as the series of dots 1106.

Pixel intensity analysis as discussed herein can include analyzing any suitable pixel characteristic, such as brightness, luminance, hue, saturation, and the like. Selecting a suitable characteristic for pixel intensity analysis may depend on characteristics of the source light, characteristics of captured light, and similar.

The process is repeated for each image frame captured, so that updates are made in real time and new veins are detected and have their midpoints projected as the series of dots 1106 while the catheter 210 is moved. The clinician can then reference the series of dots 1106 when selecting a suitable vein and then aligning the catheter 210 with the selected vein.

The discrimination and guidance unit 200-4 can further be configured to output feedback for aiding alignment of the catheter 210 with the series of dots 1106 indicating the selected vein. The feedback can be visual, audible, or a combination of such. For example, the color of the dots 1106 can be changed, an additional visual object can be projected, or the unit 200-4 can emit a sound via speaker 200-6. The feedback can be configured to change as the alignment of the catheter 210 with the series of dots 1106 approaches an optimal alignment. For example, a frequency of an audible tone changes as the alignment of the catheter 210 approaches optimal alignment. Alternatively or additionally, a visual indicator is added to the image when the alignment of the catheter 210 is within a threshold of the optimal alignment.

Alignment of the catheter 210 with the series of dots 1106 can be calculated using a linear regression calculation. Referring to Figure 13a, the distances of the points 1304 from the tracking line 1300 can be used to calculate an error (e.g., using a sum of squares of the distances). The optimal alignment can be a line of best fit. Weighting factors may be applied to the distances, such that points 1304 closer to the catheter tip 1103 contribute more to the calculated error, as such points may be more important to alignment.

For continuous feedback, such as a tone of changing frequency, the feedback can be proportional to the calculated error. For thresholded feedback, such as a visual indicator added to the image, such feedback can be provided when the calculated error is below a threshold error.

The discrimination and guidance unit 200-4 can be configured to discriminate between veins by comparing vectors between the points 1304, in that positionally distant groups of points or groups of points following different paths are treated as different veins. Thus, the techniques described above can be limited to a group of points 1304 that is determined to be nearest the catheter tip 1103. For example, the error calculation may be configured to exclude points not belonging to the group nearest the catheter tip 1103, so that alignment feedback is not unduly influenced by a vein that is not intended to be selected.

In other embodiments, as shown in Figure 11b, a compass line 1108 is included in the images projected onto the skin surface 1100 by the discrimination and guidance unit 200-4. The compass line 1108 is a visual indicator that extends from the catheter tip 1103 towards a midline 1110 of the vein 1104. The catheter direction line 1102 is included in the projected images, and the series of dots 1106 (Figure 11a) may be included or omitted.

As the clinician moves the catheter 210, as indicated by arrow M, the angle A between the catheter direction line 1102 and the compass line 1108 changes. As the angle A approaches zero, that is, as the catheter direction line 1102 and the compass line 1108 become closer, feedback may be provided, as discussed above. The clinician is thus guided to reduce the angle A and align the catheter direction line 1102 and the compass line 1108, so as to align the catheter direction line 1102 with the midline 1110 of the vein 1104.

The discrimination and guidance unit 200-4 is configured to provide the compass line 1108 according to a vein detected near the catheter tip 1103. If two or more veins are determined to be suitably near the catheter tip 1103, then the discrimination and guidance unit 200-4 selects the vein with the largest number of dark pixels.

The compass line 1108 can be configured to behave according to a conceptual center of gravity. That is, when the catheter 210 is distant to the vein 1104, the compass line 1108 points towards the nearest best (darkest) vein as if there is a gravity force present. Signal conditioning can be performed on the compass line 1108 to provide a consistent and nonvolatile presentation. A filter can be applied to a calculated angle of the compass line 1108, so that insignificant angular fluctuation is avoided.

Referring to Figure 13b, the discrimination and guidance unit 200-4 is configured to analyze a sweep area within a predetermined sweep angle B about the tracking line 1300 that extends from the catheter tip 1103. A set of sweep lines 1308 are analyzed within the area. The number of sweep lines 1308 may be selected to cover substantially all angles within the sweep angle B or to sample substantially all pixels within the sweep area.

The image pixels in each sweep line 1308 are analyzed for intensity. The discrimination and guidance unit 200-4 selects the sweep line 1308 with the greatest number of pixels with the least overall intensity as the direction for the compass line 1108. Such pixels can be determined according to various methods, such as a count of pixels of intensity below a threshold intensity, a total intensity being a normalized sum of intensities for all pixels on the sweep line, and the like.

The present invention has been described with respect to the forgoing embodiments and variations. Other embodiments and variations are possible. For example, although the preferred embodiment is discussed in connection with an infrared detector and image projector system 200, such as the VeinViewer® system manufactured and sold by Christie Medical Holdings, Inc., the principles of catheter discrimination and guidance may be implemented in HUD (head's up display) or other types of vascular imaging systems.

The many features and advantages of the invention are apparent from the detailed specification and, thus, it is intended by the appended claims to cover all such features and advantages of the invention that fall within the true spirit and scope of the invention. Further, since numerous modifications and changes will readily occur to those skilled in the art, it is not desired to limit the invention to the exact construction and operation illustrated and described, and accordingly all suitable modifications and equivalents may be resorted to, falling within the scope of the claims.

**Claims**

1. A catheter discrimination and guidance system, comprising:

    an IR imager for capturing an image of patient skin tissue, catheter and needle;
    a display; and
    a discrimination and guidance unit configured to superimpose one or more visual indicators on the image of patient skin tissue via the display for guiding insertion of the catheter toward a vein.

2. The catheter discrimination and guidance system of claim 1, wherein the one or more visual indicators comprises one or more of: a series of dots representative of the vein, a compass line extending from a tip of the catheter towards pixels of intensity indicative of the vein and a catheter direction line extending from a tip of the catheter in a direction of the needle based on a pixel intensity of the image.

3. The catheter discrimination and guidance system of claim 2, wherein the one or more visual indicators comprises the catheter direction line, and the discrimination and guidance unit is configured to perform a pixel intensity analysis of the image and to provide feedback based on one or more of: a calculated alignment of the catheter direction line to at least a subset of the series of dots and a calculated alignment of the catheter direction line to the compass line or the subset of the series of dots.

4. The catheter discrimination and guidance system of any one of claims 1 to 3, wherein the discrimination and guidance unit is configured for (i) performing a calibration operation to estimate the length of the catheter using the image, (ii) determining an orientation angle of the catheter into the tissue, and (iii) determining location of catheter and needle end point locations using the length and orientation angle, wherein the one or more visual indicators are generated using the length, orientation angle and end points.

5. The catheter discrimination and guidance system of claim 4,
wherein superimposing the one or more visual indicators includes at least one of:

   displaying the catheter end point locations relative to the vein;
   displaying colored points, lines and text information indicative of catheter location relative to the vein; and
   displaying visual indicators of vein cannulation by the catheter.

6. The catheter discrimination and guidance system of any one of claims 1 to 5:

   further adapted to calculate and project a visual indicator, $b$, indicating the location of cannulation based on the location, $a$, of needle-skin access point, said orientation angle, $\theta$, and a vein depth, $x_v$, according to $|\overrightarrow{b\text{-}a}| = |\overrightarrow{ab}| = x_v.tan(\theta)$, the vein depth being determined by reducing light intensity until vein contrast is lost.

7. A method of catheter discrimination and guidance, comprising:

   capturing an image of patient skin tissue, catheter and needle and discriminating the catheter and needle therefrom; and
   superimposing one or more visual indicators on the image of patient skin tissue for guiding insertion of the catheter toward a vein;
   wherein the one or more visual indicators comprises one or more of: a series of dots representative of the vein, a compass line extending from a tip of the catheter towards pixels of intensity indicative of the vein, and a catheter direction line extending from a tip of the catheter in a direction of the needle based on a pixel intensity of the image; and
when the one or more visual indicators comprises the catheter direction line, further comprising determining an alignment of the catheter direction line to at least a subset of the series of dots and outputting feedback based on any one of: the determined alignment and a calculated alignment of the catheter direction line to the compass line or the subset of the series of dots.

8. The method of claim 7, further comprising:

   performing a calibration operation to estimate the length of the catheter using the image;
   determining an orientation angle of the catheter into the tissue; and
   determining location of catheter and needle end point locations using the length and orientation angle;
   wherein the one or more visual indicators are generated using the length, orientation angle and end points.

9. The catheter discrimination and guidance system of claim 7 or claim 8, further including determining the depth of the vein using tissue thickness and associated reflectance information, said determining the depth of the vein comprises reducing light intensity until vein contrast is lost.

10. The method of any one of claims 7 to 9, wherein superimposing the one or more visual indicators includes at least one of:

    displaying the catheter end point locations relative to the vein;
    displaying colored points, lines and text information indicative of catheter location relative to the vein; and
    displaying a map of veins adjacent the location of vein cannulation.

11. The method of any one of claims 8 to 10, wherein the calibration operation comprises obtaining images of the catheter prior to tissue insertion as the needle is rotated through positive and negative angles relative to the surface of the patient skin tissue, and identifying actual length of the catheter as the maximum observed length at zero degrees to the surface of the patient skin tissue.

12. The method of any one of claims 7 to 11, further comprising discriminating the catheter and needle from the image by frequency filtering and thereafter analyzing the filtered image to locate the pixel having either minimum intensity value in the event the needle forms part of a translucent catheter that is permeable to infrared light or maximum intensity value in the event the catheter forms part of an opaque catheter, and using the value as a seed point to detect catheter contour pixels with similar intensities.

13. The method of any one of claims 7 to 12, further comprising the step of thresholding the image of the catheter about the intensity of the seed point, applying a line-fit to the thresholded image to identify movement direction of the catheter and defining its end points; and identifying the start point of the needle within tissue by a sharp drop in intensity and identifying the catheter tip cannulating the vein by disappearance of the sharp drop in intensity.

14. The method of any one of claims 7 to 13, further comprising calculating and projecting a visual indicator, $b$, indicating the location of cannulation based on the location, $a$, of needle-skin access point, the orientation angle, $\theta$, and the determined vein depth, $x_v$, according to $|\overrightarrow{b\text{-}a}| = |\overrightarrow{ab}| = x_v.tan(\theta)$.

15. The method of claim 14, further comprising projecting one or more of: coloured dots at locations $a$ and $b$ for guiding insertion of the needle toward the location of cannulation and perpendicular lines to the catheter movement direction at locations $a$ and $b$.

Incident Beam

**FIG. 1**

**FIG. 2**

**FIG. 3**

325

$I_n$

θ

−θ

$I_n - COS(θ)$

$I_n - COS(0)$

**FIG. 4**

a)

b)

c)

**FIG. 5**

a)

Intensity Along the needle

Intensity Change within tissue

Catheter Start Point

Catheter Observed Length

Catheter End Point

Needle

Distance Along the needle

b)

**FIG. 6**

**210**

Start point

End point

**210-2**

Observed
Length

Angle

**210-3**

**210-1**

**210-4**

**Hexagonal
housing feature**

## FIG. 7

Catheter and
housing Image

Feature
Extraction —— 800-1

800-5 —— Sample
Feature

Correlation
Analysis —— 800-3

Classification —— 800-7

Vendor
Selection —— 800-9

## FIG. 8

**210-2**

**225**

Incident Light Beam

Catheter

Ic

θ

Vein Skin

$X_v$

**220**

**215**

a)

Change direction of the illuminated point

**210-2**

θ

Ic

$I_c \cdot \cos(\theta)$

θ

a

θ $X_n$

$X_v$

b

The camera view

b

a

Catheter

**220**

Tissue

Vein

**215**

$X_v \cdot \tan(\theta)$

b)

**FIG. 9**

**FIG. 10**

M

1100

1106

1103  1102

1104

210

**FIG. 11a**

1102

1100

1110

1108

A

M

1103

210

1104

**FIG. 11b**

**FIG. 12a**

**FIG. 12b**

**FIG. 13a**

**FIG. 13b**

Europäisches Patentamt

European Patent Office

Office européen des brevets

# EUROPEAN SEARCH REPORT

Application Number

EP 13 18 7961

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/125028 A1 (WOOD FRED [US] ET AL) 26 May 2011 (2011-05-26) | 1,2,7 | INV.<br>A61B5/00 |
| Y | * paragraph [0003] *<br>* paragraph [0084] - paragraph [0085] *<br>* paragraph [0102] - paragraph [0103] *<br>* paragraph [0105] *<br>* figure 70 *<br>* paragraph [0112] *<br>* figure 14 * | 12 | A61B17/34<br>A61B19/00 |
| X,D | US 2006/122515 A1 (ZEMAN HERBERT D [US] ET AL) 8 June 2006 (2006-06-08) | 1,2,7 | |
| Y | * paragraph [0041] *<br>* figure 24 *<br>* paragraph [0044] - paragraph [0045] * | 9 | |
| X | WO 2011/063266 A2 (UNIV JOHNS HOPKINS [US]; STOLKA PHILIPP JAKOB [US]; BOCTOR EMAD MOUSSA) 26 May 2011 (2011-05-26) | 1,2,4,<br>6-8,11,<br>13-15 | |
| Y | * paragraph [0056] *<br>* paragraph [0063] *<br>* paragraph [0106] *<br>* figure 7 *<br>* figure 8 * | 5 | TECHNICAL FIELDS SEARCHED (IPC)<br><br>A61B |

-/--

The present search report has been drawn up for all claims

| Place of search | Date of completion of the search | Examiner |
|---|---|---|
| The Hague | 20 December 2013 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 13 18 7961

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/029521 A2 (BEN CHORIN MOSHE [IL]; WEISS NIR [IL]; LEVIN GUY [IL]; LEVIN ORI [IL]) 18 March 2010 (2010-03-18) | 1-3,7, 10,15 | |
| Y | * figure 5D *<br>* figure 14 *<br>* figure 13 *<br>* page 6, line 3 - line 19 *<br>* page 8, line 6 - line 11 *<br>* page 14, line 5 - line 9 *<br>* page 22, line 27 - page 23, line 5 *<br>* page 41, line 16 - page 42, line 10 *<br>* page 50, line 9 - line 17 *<br>* page 50, line 30 - page 51, line 3 *<br>* page 51, line 8 - line 15 *<br>* page 51, line 20 - line 24 * | 5 | |
| X | WO 2006/073869 A2 (SYRIS SCIENT LLC [US]; MARCOTTE RONALD [US]; ARSENAULT MARK [US]; HEBO) 13 July 2006 (2006-07-13)<br>* figure 3 *<br>* figure 5c *<br>* page 5, line 8 - line 17 *<br>* page 5, line 18 - line 28 *<br>* page 8, line 22 - page 9, line 9 * | 1-3,7,10 | |
| X | WO 2006/131881 A1 (KONINKL PHILIPS ELECTRONICS NV [NL]; SCHWACH CAROLE [DE]; NEERKEN SIEG)<br>14 December 2006 (2006-12-14)<br>* page 4, line 14 - page 6, line 10 *<br>* figure 5 *<br>* figure 6 * | 1,2,7 | |

TECHNICAL FIELDS
SEARCHED     (IPC)

-/--

The present search report has been drawn up for all claims

| Place of search | Date of completion of the search | Examiner |
|---|---|---|
| The Hague | 20 December 2013 | Weiss-Schaber, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
    document

EPO FORM 1503 03.82 (P04C01)

| | Europäisches Patentamt European Patent Office Office européen des brevets | **EUROPEAN SEARCH REPORT** | Application Number EP 13 18 7961 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Ganesh, Soujanya: "DEPTH AND SIZE LIMITS FOR THE VISIBILITY OF VEINS USING THE VEINVIEWER IMAGING SYSTEM", , 1 May 2007 (2007-05-01), XP002718250, Retrieved from the Internet: URL:http://etd.uthsc.edu/WORLD-ACCESS/ganesh/2007-001-ganesh.pdf [retrieved on 2013-12-11] * 5.4.1 Contrast-Depth Correlation * ----- | 9 | |
| Y | US 2001/048077 A1 (AFANASSIEVA NATALIA I [US]) 6 December 2001 (2001-12-06) * the whole document * ----- | 12 | |
| | | | TECHNICAL FIELDS SEARCHED (IPC) |

The present search report has been drawn up for all claims

| Place of search | Date of completion of the search | Examiner |
|---|---|---|
| The Hague | 20 December 2013 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 13 18 7961

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2013

| Patent document cited in search report | | | Publication date | Patent family member(s) | | | Publication date |
|---|---|---|---|---|---|---|---|
| US 2011125028 | A1 | | 26-05-2011 | US | 2011125028 | A1 | 26-05-2011 |
| | | | | WO | 2012011951 | A1 | 26-01-2012 |
| US 2006122515 | A1 | | 08-06-2006 | NONE | | | |
| WO 2011063266 | A2 | | 26-05-2011 | CA | 2781427 | A1 | 26-05-2011 |
| | | | | EP | 2501320 | A2 | 26-09-2012 |
| | | | | JP | 2013511355 | A | 04-04-2013 |
| | | | | US | 2012253200 | A1 | 04-10-2012 |
| | | | | US | 2013016185 | A1 | 17-01-2013 |
| | | | | WO | 2011063266 | A2 | 26-05-2011 |
| WO 2010029521 | A2 | | 18-03-2010 | NONE | | | |
| WO 2006073869 | A2 | | 13-07-2006 | EP | 1841361 | A2 | 10-10-2007 |
| | | | | EP | 2412313 | A1 | 01-02-2012 |
| | | | | US | 2006173351 | A1 | 03-08-2006 |
| | | | | WO | 2006073869 | A2 | 13-07-2006 |
| WO 2006131881 | A1 | | 14-12-2006 | CN | 101193595 | A | 04-06-2008 |
| | | | | EP | 1893093 | A1 | 05-03-2008 |
| | | | | JP | 2008545502 | A | 18-12-2008 |
| | | | | US | 2008221519 | A1 | 11-09-2008 |
| | | | | WO | 2006131881 | A1 | 14-12-2006 |
| US 2001048077 | A1 | | 06-12-2001 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 13648517 B
- US 13865650 B
- US 7239909 B
- US 2010051808 A
- US 20070158569 A
- US 20060122515 A

**Non-patent literature cited in the description**

- **ANDERSON, R.R. ; PARRISH, J.A.** *The Journal of Investigative Dermatology,* 1981, vol. 77, 13-19
- **BARRON, V. ; TORRENT, J.** *Journal of Soil Science,* 1986, vol. 37, 499-510
- **BASHKATOV, A.N. et al.** *Journal of Physics D: Applied Physics,* 2005, vol. 38, 2543-2555
- **EDSTOROM, P. et al.** *Nordic Pulp and Paper Research Journal,* 2010, vol. 25, 221-232
- **IVANOV, A.P. ; BARUN, V.V.** *Journal of Engineering Physics and Thermophysics,* 2011, vol. 84, 23-32
- **KUBELKA, P.** *Journal of the Optical Society of America,* 1948, vol. 38, 448-457
- **KUBELKA, P. ; MUNK, F.** *Zeitschrif fur technische Physik,* 1930, vol. 31, 1-16
- **NEUMAN, M. ; EDSTROM, P.** *Journal of the Optical Society of America,* vol. 27, 1040-1045
- **NEUMAN, M. ; EDSTROM, P.** *Journal of the Optical Society of America,* vol. 27, 1032-1039
- **NEUMAN, M. ; COPPEL, L.G. ; EDSTROM, P.** *Optics Express,* 2011, vol. 19, 1915-1920
- **PREMOZE, S.** *Proceedings of the 10th Pacific Conference on Computer Graphics and Applications,* 2002, 48-58
- **ROGGAN, A. et al.** *Journal of Biomedical Optics,* 1999, vol. 4, 35-46
- **SCHWEIGER, M. et al.** *Americal Association of Physicist in Medicine,* 1995, vol. 22, 1779-1792
- **AYVACI, A. et al.** *Computerized Medical Imaging and Graphics,* 2011, vol. 35, 653-659
- **DONG, B. ; SAVITSKY, E. ; OSHER, S.** *Proceedings of the 5th International Symposium on Advances in Visual Computing: Part I,* 2009, 1-9
- **HONG, J. et al.** *A motion adaptable needle placement instrument based on tumor specific ultrasonic image segmentation,* 2002, 122-129
- **KAVITHA, R. ; FLOWER, L.** *International Journal of Engieering Science and Technology,* 2011, vol. 3, 4833-4838
- **NAJAFI, M. ; ROHLING, R.** *SPIE Proceedings of Visualization, Image-Guided Procedures, and Modeling,* 2011, vol. 7964, 79641F
- **OKAZAWA, S.H. et al.** Methods for segmenting cuved needles in ultrasound images. *Medical Image Analysis,* 2006, vol. 10, 330-342
- **PAQUIT, V. et al.** *SPIE Proceedings of Visualization, Image-Guided Procedures, and Display,* 2006, vol. 6141, 61411T
- **SZNITMAN, R. et al.** *Proceedings of Medical Image Computing and Computer-Assisted Intervention,* 2011, 1-8